# EUROPEAN PATENT APPLICATION

(11) **EP 4 501 347 A1**
(43) Date of publication of application: **05.02.2025**
(21) Application number: 23189239.9
(22) Date of filing: 02.08.2023
(51) Int. Cl.: A61K 38/38, A61P 25/00

(54) **PEPTIDES FOR USE IN THE TREATMENT OF CENTRAL NERVOUS SYSTEM DISORDERS**

(71) Applicant: Forssmann, Wolf-Georg, 79692 Kleines Wiesental (DE)
(72) Inventor: Forssmann, Wolf-Georg, 79692 Kleines Wiesental (DE)
(74) Representative: dompatent von Kreisler Selting Werner - Partnerschaft von Patent- und Rechtsanwälten mbB

(57) **Abstract**

A peptide for use in treating and/or preventing central nervous disorders, wherein the peptide is selected from the group consisting of the following:
(i) a peptide having the following amino acid sequence:
Z₁-LVRYTKKVPQVSTPTL-Z₂ (ALB-408);
(ii) peptides obtainable by multiple synthesis which have the biological activity of ALB408-423;
and
(iii) biologically active fragments and/or variants and/or derivatives, especially PEGylated, HESylated, amidated, acetylated, sulfated, phosphorylated and/or glycosylated derivatives, of (i) and (ii);
wherein Z represents a number of from 0 to 10 amino acid residues.

## Description

### Field of the invention

The present invention relates to peptides for use in treating and/or preventing of Central Nervous System Disorders, a pharmaceutical composition for use in treating and/or preventing Central Nervous System Disorders as well as a method for treating Central Nervous System Disorders in a subject in need thereof. The peptides for use of the present invention are CXC chemokine receptor 4 (CXCR4) antagonistic peptides (proteins). The peptides for use of the present invention include the naturally occurring form of human circulating antiviral albumin fragment (ALB408-423), peptides obtainable by multiple synthesis which have the biological activity of ALB408-423, and biologically active fragments and/or variants and/or derivatives of the aforementioned.

### Background of the invention

Central nervous disorders encompass several different disorders, such as neurodegeneration, chronic pain, stroke, spinal cord injury, and many other CNS diseases. The delivery of drugs to the brain is challenging, as peptides which would act in the brain, usually have a low metabolic stability. This usually results in the need of parenteral administration, such as intravenous, intramuscular etc.

Theses ways of administration decrease the acceptance of peptides in the therapy and limit their use, as they can only be administered by a doctor and not by a patient himself/herself.

Therefore, there exists a need to develop further possibilities of applying suitable compounds and medicaments for use in the treatment and/or prevention of central nervous disorders.

Chemokine receptors are expressed on the surface of certain cells, which interact with cytokines called chemokines. The CXC chemokine receptor 4 (CXCR4) is a G-protein-coupled receptor that transduces signals of its endogenous ligand, the chemokine CXCL12 (stromal cell-derived factor-1, SDF-1). Following interaction of CXCR4/CXCL12 intracellular calcium (Ca²⁺) ions fluxes are triggered. This causes cellular responses, including chemotaxis allowing cells to travel within the organism. CXCR4 is expressed on myeloid cells, T-lymphocytes, B-lymphocytes, epithelial cells, endothelial cells and dendritic cells. The chemokine CXCL12 is the only known agonistic ligand of CXCR4. The interaction between CXCL12 and CXCR4 plays a crucial role in the migration of progenitor cells during embryologic development of the cardiovascular, hemopoietic or central nervous systems. This interaction is also known to be involved in several diseases such as HIV infection/AIDS, cancer cell metastasis, leukemia cell progression, pulmonary fibrosis and rheumatoid arthritis. It is assumed that this interaction may be a critical therapeutic target in all of these diseases. Substances interfering with CXCR4/CXCL12 signaling are assumed to have drug potential, e.g., in HIV/AIDS therapy, or to prevent cell migration processes involved in cancer metastasis, leukemia, and inflammatory diseases such as pulmonary fibrosis, rheumatoid arthritis or asthma (reviewed in Tsutsumi et al., 2007, Peptide Science 88: 279 - 289). In contrast to receptor agonists such as CXCL12 that induce cellular responses, receptor antagonists are ligands or drugs that do not induce a biological response, i.e., cell migration or Ca²⁺ signaling, upon binding to their receptor. Receptor antagonists are useful drugs already in clinical use (e.g., angiotensin antagonists, β-adrenergic antagonists, serotonergic antagonist or CCR5 antagonists) that can block HIV-1 infection (CCR5 antagonist) or decrease agonist mediated cellular responses. Interaction of receptor antagonists with the receptor inhibits the function of an agonist. Most drug antagonists achieve their potency by competing with endogenous ligands or substrates at structurally defined binding sites on receptors.

WO 2009/004054 A2 discloses that the chemokine receptor CXCR4 is a therapeutic target for the treatment of HIV/AIDS, cancer associated pathologies and chronic inflammatory diseases like asthma or pulmonary fibrosis. The application discloses a peptide having the amino acid sequence Z₁-LVRYTKKVPQVSTPTL-Z₂ (ALB-408), its biologically active fragments and/or variants and/or derivatives and peptides obtainable by multiple synthesis which have the biological activity of ALB408-423, as well as their use in the preparation of a medicament for the treatment of viral diseases, bacterial and fungal infections, inflammatory processes, disturbed inflammation reactions, tumor diseases, growth disorders, neuronal diseases, diseases of blood clotting and hematopoiesis, vascular diseases, diseases of the immune system, and for wound and bone healing.

WO 2014/198834 A1 discloses peptides, in particular dimers, effective in blocking the CXCR4 mediated HIV-1 NL4-3 (X4-tropic) infection with an IC₅₀ value of less than 50 µM.

WO 2019/048666 A1 discloses peptides for use in the treatment of cancers, in particular cancers showing the CRCX receptor such as cancer of the liver, pancreas, prostate, or breast cancer.

WO 2020/094843 A1 discloses polypeptides for use in the treatment of cancers, in particular cancers showing the CRCX receptor such as cancers of the liver, pancreas, prostate, or breast cancer.

An object of the invention is to provide peptides for use in treating and/or preventing central nervous disorders. Especially, the peptides should be applicable in a way which is possible for the patient himself/herself.

A further object of the invention is to provide a pharmaceutical composition for use in treating and/or preventing central nervous disorders.

A still further object of the present invention is to provide a method for treating central nervous disorders in a subject in need thereof.

### Summary of the invention

Unexpectedly, it was found that the CXCR4 antagonistic polypeptides (i) having the amino acid sequence Z₁-LVRYTKKVPQVSTPTL-Z₂ (ALB-408), wherein Z represents a number of from 0 to 10 amino acid residues, (ii) peptides obtainable by multiple synthesis which have the biological activity of ALB408-423, and (iii) biologically active fragments and/or variants and/or derivatives, especially PEGylated, HESylated, amidated, acetylated, sulfated, phosphorylated and/or glycosylated derivatives of the aforementioned, can be effectively used in a method of treating and/or preventing central nervous disorders.

The present invention therefore pertains to a peptide for use in treating and/or preventing central nervous disorders, wherein the peptide is selected from the group consisting of the following:
(i) a peptide having the following amino acid sequence:
   Z₁-LVRYTKKVPQVSTPTL-Z₂ (ALB-408);
(ii) peptides obtainable by multiple synthesis which have the biological activity of ALB408-423;
   and
(iii) biologically active fragments and/or variants and/or derivatives, especially PEGylated, HESylated, amidated, acetylated, sulfated, phosphorylated and/or glycosylated derivatives, of (i) and (ii);
wherein Z represents a number of from 0 to 10 amino acid residues.

The peptide for use of the present invention may be a peptide for use of the invention, wherein single or several amino acid residues in the sequence have been exchanged, deleted, or added, or wherein chemical modifications on single amino acids of the peptides for use of the invention have been introduced which have similar or the same biological or pharmacological activity of the peptides for use of the invention. In particular, those peptides are concerned which can easily be obtained by exchanging amino acids of the sequence in a conservative manner which means to exchange hydrophobic amino acids against hydrophobic ones or aromatic against other aromatic amino acids or basic amino acids against other basic amino acids and the like. This is well known to the skilled person.

Also, retro-inverso peptides of the peptides for use of the invention are in the scope of the present invention, as well as other derivatives stabilizing the peptide bond against peptidases.

The term "derivative" means all length fragments including truncations at the N and C terminus, peptides containing amino acid residue substitutions including D-amino acid residues and modified amino acid residues as well as peptides containing disulfide bonds and extension at the N and C terminus, and peptides, wherein at least one side chain of an amino acid of said peptide is chemically modified. Especially, the term "derivative" means PEGylated, HESylated, amidated, acetylated, sulfated, phosphorylated and/or glycosylated derivatives of the peptides (i) and (ii) for use of the invention.

The term "variants" means peptides having an amino acid sequence having at least 70% sequence identity, preferably at least 80% sequence identity, more preferably at least 90% sequence identity, and most preferably at least 95% sequence identity, to any one of the peptides (i) and (ii) for use of the invention. The terms "sequence identity" or "percent sequence identity" in the context of two or more peptides refer to two or more sequences or subsequences that are the same or have a certain percentage of amino acids that are identical when compared and "aligned" for maximum similarity using a sequence comparison algorithm. Optimized alignment for comparing sequences can be performed using, for example, the following algorithms: Local Homology Alignment of Smith & Waterman, Adv. Appl. Math. 2: 482 (1981), Homology Alignment Algorithm by Needleman & Wunsch, J. Mol.Biol. 48: 443 (1970), Pearson & Lipman, Proc. Nat'l. Acad.Sci. USA 85: 2444 (1988), (GAP, BESTFIT, FASTA, and TFASTA in Wisconsin Genetics Software Package, Genetics Computer Group, 575 Science Dr., Madison, WI), but also visual examination (compare, Current Protocols in Molecular Biology, (Ausubel, F. M. et al., eds.) John Wiley & Sons, Inc, New York (1987-1999, including Supplement 46 (April 1999)).

The peptides obtainable by multiple synthesis which have the biological activity of ALB408-423 may be the peptides selected from the group consisting of SEQ ID NOs: 1 to 31 and/or the peptides having the general amino acid sequence written in the single letter code
X¹ X² R X⁴ X⁵ X⁶ K X⁸ P X¹⁰ X¹¹ S,
wherein
X¹ = Lor I,
X² = V, M, or L,
X⁴ = Y or W,
X⁵ = T or S,
X⁶ = K, C, R, or Q,
X⁸ = V, M, L, F, or A,
X¹⁰ = Q, or C,
X¹¹ = V, M, or F.

Preferably, the peptides obtainable by multiple synthesis which have the biological activity of ALB408-423 are the peptides selected from the group consisting of SEQ ID NOs: 1 to 31 and/or the peptides having the general amino acid sequence written in the single letter code
X¹ V R X⁴ X⁵ X⁶ KV P X¹⁰ V S,
wherein
X¹ = L or I,
X⁴ = Y or W,
X⁵ = T or S,
X⁶ = K or C,
X¹⁰ = Q or C.

In another preferred embodiment, the peptides obtainable by multiple synthesis which have the biological activity of ALB408-423 are the peptides having the general amino acid sequence written in the single letter code
X¹ X² R X⁴ X⁵ X⁶ K X⁸ P X¹⁰ X¹¹ S,
wherein
X¹ = Lor I,
X² = V, M, or L,
X⁴ = Y or W,
X⁵ = T or S,
X⁶ = K, C, R, or Q,
X⁸ = V, M, L, F, or A,
X¹⁰ = Q, or C,
X¹¹ = V, M, or F;
preferably the peptides having the general amino acid sequence written in the single letter code
X¹ V R X⁴ X⁵ X⁶ KV P X¹⁰ V S,
wherein
X¹ = L or I,
X⁴ = Y or W,
X⁵ = T or S,
X⁶ = K or C,
X¹⁰ = Q or C.

In another preferred embodiment, the peptide for use of the invention is selected from the group consisting of a peptide having the following amino acid sequence:
IVRYTKKVPQVS (SEQ ID NO: 21),
and its amidated, acetylated, sulfated, phosphorylated and/or glycosylated derivatives.

In another preferred embodiment, the peptide for use of the invention has the following amino acid sequence:
IVRYTKKVPQVS (SEQ ID NO: 21).

The central nervous disorders may be selected from the group consisting of neurodegeneration, chronic pain, stroke, spinal cord injury, Alzheimer's disease, Bell's palsy, cerebral palsy, epilepsy, motor neurone disease, multiple sclerosis, neurofibromatosis, parkinsons's disease, sciatice or shingles.

In a preferred embodiment, the peptide is administered intranasal. Surprisingly it was found that an intranasal administration effectively allows treatment of central nervous disorders.

The peptide for use of the invention may be administered two or three times a day, preferably three times a day, for one to three months, preferably for four to six weeks. The treatment duration may be extended as needed.

In a preferred embodiment, the peptide is administered on the intranasal route, three times a day, preferably on the intranasal route, three times a day, for six weeks.

Preferably, the peptide for use of the invention is administered on the intranasal route with a nasal spray.

The peptide for use may be administered in a concentration between 0.5 mg/mL and 2.0 mg/mL, preferably in a concentration of 1.0 mg/mL.

The peptide for use may be provided in a sodium chloride solution, preferably a physiological sodium chloride solution, or in a citrate/bicarbonate buffer, or in a citrate/phosphate buffer, preferably in a citrate/phosphate buffer. Suitable buffers according to Pharmacopoeia Europaea (Ph. Eur.) are known to the person skilled in the art. The concentration of the peptide for use may be between 0.5 mg/mL and 2.0 mg/mL, preferably 1.0 mg/mL.

In a preferred embodiment, the invention pertains to the peptide having the amino acid sequence IVRYTKKVPQVS (SEQ ID NO: 21) for use in treating and/or preventing osteosarcoma.

Another subject of the present invention is a pharmaceutical composition for use in treating and/or preventing central nervous disorders, wherein the pharmaceutical composition comprises or consists of
(a) a peptide having the following amino acid sequence:
   Z₁-LVRYTKKVPQVSTPTL-Z₂ (ALB-408),
   peptides obtainable by multiple synthesis which have the biological activity of ALB408-423, and biologically active fragments and/or variants and/or derivatives, especially PEGylated, HESylated, amidated, acetylated, sulfated, phosphorylated and/or glycosylated derivatives thereof;
   wherein Z represents a number of from 0 to 10 amino acid residues; and
(b) a pharmaceutically acceptable carrier.

The pharmaceutical composition for use comprises the peptides for use of the present invention. Therefore, it is to be understood that all the embodiments and features described herein with regard to the peptides for use of the invention apply accordingly to the pharmaceutical composition for use of the invention.

In a preferred embodiment, the peptide has the following amino acid sequence:
IVRYTKKVPQVS (SEQ ID NO: 21).

The pharmaceutical composition for use may be suitable for intranasal, administration.

The pharmaceutical composition for use may be in the form of a liquid preparation.

The concentration of the peptide may be between 0.5 mg/mL and 2.0 mg/mL, preferably 1.0 mg/mL.

In a preferred embodiment, the pharmaceutically acceptable carrier is a sodium chloride solution, preferably a physiological sodium chloride solution, or a citrate/bicarbonate buffer, or a citrate/phosphate buffer. Suitable buffers according to Pharmacopoeia Europaea (Ph. Eur.) are known to the person skilled in the art. The concentration of the peptide in the pharmaceutically acceptable carrier may be between 0.5 mg/mL and 2.0 mg/mL, preferably 1.0 mg/mL.

A still further subject of the present invention is a method for treating central nervous disorders in a subject in need thereof comprising, administering to the subject an effective amount of a peptide, wherein the peptide is selected from the group consisting of the following:
(i) a peptide having the following amino acid sequence:
   Z₁-LVRYTKKVPQVSTPTL-Z₂ (ALB-408);
(ii) peptides obtainable by multiple synthesis which have the biological activity of ALB408-423; and
(iii) biologically active fragments and/or variants and/or derivatives, especially PEGylated, HESylated, amidated, acetylated, sulfated, phosphorylated and/or glycosylated derivatives, of (i) and (ii);
wherein Z represents a number of from 0 to 10 amino acid residues.

In the method for treating central nervous disorders of the present invention, the peptide administered in an effective amount is the peptide for use of the present invention. The peptide may also be administered in the form of the pharmaceutical composition for use of the present invention. Therefore, it is to be understood that all the embodiments and features described herein with regard to the peptides for use of the invention and the pharmaceutical composition for use of the invention apply accordingly to the method for treating central nervous disorders of the present invention.

The peptide for use of the invention can be provided by a process comprising an extraction from hemofiltrate by cation-exchange extraction followed by elution of adsorbed substances, renewed cation-exchange chromatography of the extract containing the peptides, and fractional reverse-phase chromatography.

Alternatively, the process for the manufacturing of the peptides for use according to the invention can be performed by solid-phase synthesis in terms of a Merrifield synthesis or liquid-phase synthesis by methods known per se to the skilled person using protected amino acids, and its purification.

A further process for the manufacturing of the peptides for use according to the invention employs methods of heterologous expression known to the skilled person using common biotechnological vectors.

The invention is described in more detail using the peptide IVRYTKKVPQVS (SEQ ID NO: 21) as specific example. It is readily understood that likewise the peptides for use of the invention can replace IVRYTKKVPQVS (SEQ ID NO: 21) in the following descriptions.

### Detailed description of the invention

ALB408-423 could be isolated from human hemofiltrate by means of chromatographical methods and a biological assay. The biochemical characterization of the peptide was effected by mass spectrometry including a complete sequence analysis of the amino acids.

The peptide has the following amino acid sequence SEQ ID NO: 8:
LVRYTKKVPQVSTPTL

The molecular weight of the peptide ALB408-423 is 1830.2 Da. The isoelectric point (pI) of the peptide ALB408-423 is 10.3.

The peptide ALB408-423 is a fragment comprising 16 amino acids of the known human plasma protein serum albumin (Accession No. NP000468), which consists of 585 amino acids in its processed form. Human albumin is a soluble monomeric serum protein having a molecular weight of about 65,000 that accounts for more than half the total plasma protein (concentration: 3.5 to 5 g/dL). The function of human albumin is predominantly described as a carrier molecule for all kinds of hydrophobic as well as hydrophilic substances, e.g., steroid and peptide hormones, fatty acids, vitamins, pharmaceuticals and cations. Due to its very high serum concentration, it contributes substantially to the stabilization of the blood pH, the extracellular liquid volume and the maintenance of colloid-osmotic pressure. Albumin has a globular structure stabilized by a high number of disulfide bridges and is usually not glycosylated, but alterations due to acetylation, enzymatic glycosylation and non-enzymatic glycosylation occur frequently in the course of molecular ageing or upon pathophysiological changes. It is synthesized by the liver as pre-pro-albumin having 609 amino acids; the N-terminal signal peptide comprising 18 amino acids is cleaved of intracellularly upon entry in the endoplasmic reticulum; another 6 amino acids are removed in the Golgi apparatus before the mature albumin comprising 585 amino acids is secreted by the liver cells. The clearance of albumin takes place through the kidney, the gastrointestinal tract and in the tissue cells of the liver.

The peptide sequence of ALB408-423 for use according to the invention starts with amino acid 408 and thus comprises the amino acids 408 to 423 of the circulating form of albumin. It is evidently produced by natural processing of the albumin precursor by corresponding proteases.

The peptide ALB408-423 for use according to the invention is obtainable by chromatographic purification from human hemofiltrate (HF). HF is obtained in large amounts during ultrafiltration of the blood of kidney patients (Example 1). HF contains all peptides and proteins circulating in human blood with a molecular weight below 30 kDa. Peptides and proteins in HF were extracted using cation exchange chromatography. Column bound peptides and proteins were eluted with buffers systems of various pH values and eluates were subjected to reversed phase chromatography (Example 1). After four rounds of purification, mass spectroscopy of the active fraction 31 revealed a single peptide with a molecular weight of 1830 Da (Example 1). Sequence analyses resulted in the identification of LVRYTKKVPQVSTPTL and sequence comparison showed a 100% homology to the highly abundant serum protein "Human Serum Albumin; (ALB)" encompassing amino acid residues 408-423 (ALB408-423).

Preferred peptides for use of the invention obtainable by multiple synthesis which have the biological activity of ALB408-423 are 12-mer derivatives of the 16-mer peptide ALB408-423. Starting with the C terminal truncation of ALB408-423 (LVRYTKKVPQVS; SEQ ID NO: 12), subsequent peptides were generated through amino acid substitutions. Firstly, leucine was replaced with isoleucine resulting in IVRYTKKVPQVS (SEQ ID NO: 21). Then, tyrosine in SEQ ID NO: 21 was replaced with tryptophan resulting in IVRWTKKVPQVS (SEQ ID NO: 32). Replacement of lysine with cysteine in SEQ ID NO: 32 resulted in IVRWTCKVPQVS (SEQ ID NO: 33). Substitution of threonine, cysteine, and glutamine in SEQ ID NO: 33 by serine, lysine and cysteine, respectively resulted in IVRWSKKVPCVS (SEQ ID NO: 34). Further 12-mer derivatives of ALB408-423 derived from the abovementioned derivatives are shown in Table 1 (Example 4; SEQ ID NOs: 35 to 45).

Further peptides for use of the invention obtainable by multiple synthesis which have the biological activity of ALB408-423 are the peptides of SEQ ID NOs: 1 to 7, 9 to 11, 13 to 20, 22 to 24, and 25 to 31, which are shown in Table 1 (Example 4).

The peptides for use according to the invention are antagonists for the CXC chemokine receptor 4 (CXCR4).

Unless otherwise stated, all volumes are measured at 23 °C.

The invention will now be further described by means of the following Examples.

### Example 1

### Isolation of ALB408-423 from human hemofiltrate

Human hemofiltrate is optionally diluted with water and acidified. The pH value is preferably from 1.5 to 3.5, especially from 2.5 to 3.0. Thereafter, the hemofiltrate is passed through a cation exchanger, for example, a support material modified with sulfonic acid groups (Fraktogel SP-650 (M), Merck, Darmstadt, Germany). The peptides bound to the cation exchanger are eluted with a relatively high concentration of a salt solution. The ionic strength of the eluate is about that of a 0.5 to 1 M ammonium acetate solution.

The collected eluate is subjected to another cation exchange chromatography. This chromatography is preferably a fractional elution with buffers having increasing pH values.

The fractions containing the peptide according to the invention are further purified by preparative reverse-phase chromatography followed by semipreparative reverse-phase chromatography, for example, on C18-modified support materials. The degree of purification is preferably monitored using analytical reverse-phase chromatography, for example, on C18-modified support materials.

### 1st step: hemofiltrate batch extraction

From 800 to 1,000 liters of hemofiltrate is adjusted to a pH value of 2.7 with HCl and diluted with water to a conductivity of 5.5 mS/cm, and charged onto a strong cation exchanger with a flow rate of 3 l/min.

### Chromatographic conditions:

| | |
|---|---|
| Column: | Vantage VA 250 (Amicon, Witten, Germany) |
| Column material: | Fractogel TSK SP 650 (M), 25 cm x 20 cm |
| Flow rate: | 3 l/min |
| Detection: | 280 nm, pH, conductivity |
| Buffer A: | Hemofiltrate pH 2.7, conductivity 5.5 mS/cm |
| Buffer B: | 0.5 M ammonium acetate |

### Equipment: Autopilot Chromatographic System (PerSeptive Biosystems, Wiesbaden, Germany)

After charging the total of 1,000 liters of liquid over night, rinsing is effected with several column volumes of 5 mM HCl. The elution of the bound peptides is effected as a batch elution with 0.5 M ammonium acetate. A complete elution of the peptides is achieved through a ramping pH value (6.8 to 7.2) and ramping conductivity (56 mS/cm) in about 5 liters of eluate.

### 2nd step: First preparative separation (Batch 01/2003)

The ammonium acetate eluates of the batch extraction are combined in an amount of 10,000 liters of hemofiltrate peptide. After adjusting the pH to 2.7, the peptide extract is charged onto the preparative cation exchanger with the addition of completely desalted water having a conductivity of 5.5 mS/cm.

### Chromatographic conditions:

| | |
|---|---|
| Column: | Vantage 250 VA |
| Column material: | Fractogel TSK SP 650 (M), 25 cm x 20 cm |
| Flow rate: | up to 3 l/min during the charging |
| | 0.5 to 1 l/min during elution |
| Detection: | 280 nm, pH, conductivity |
| Sample: | Hemofiltrate pH 2.7, conductivity 5.5 mS/cm |

### Equipment: Autopilot Chromatographic System (PerSeptive Biosystems, Wiesbaden, Germany)

After charging the raw extract over 240 min, the column is rinsed with 0.01 M HCl until the conductivity is below 1 mS/cm. Elution is performed in several steps with the buffers stated below.

| Buffer | pH value | Buffer substances | Conductivity (mS/cm) |
|---|---|---|---|
| Washing buffer | 2.0 | 0.01 M HCl | 1 |
| Elution buffer 1 | 3.6 | 0.1 M citric acid monohydrate | 2.9 |
| Elution buffer 2 | 4.5 | 0.1 M acetic acid + 0.1 M sodium acetate | 4.0 |
| Elution buffer 3 | 5.0 | 0.1 M malic acid | 6.2 |
| Elution buffer 4 | 5.6 | 0.1 M succinic acid | 6.1 |
| Elution buffer 5 | 6.6 | 0.1 M NaH₂PO₄ | 4.9 |
| Elution buffer 6 | 7.4 | 0.1 M NaH₂PO₄ | 6.7 |
| Elution buffer 7 | 9.0 | 0.1 M ammonium carbonate | 6.7 |

Eluates 1-7 are designated as pH pool I-VII. They are separately collected and finally rinsed with completely desalted water. Elution is effected until a new base line is reached, elution volumes of from 10 to 25 liters being reached for the individual pH pools I to VII.

### 3rd step: Second preparative separation:

The individual pH pools are separated by reverse-phase chromatography for fractionating and simultaneous desalting.

### Chromatographic conditions:

| | |
|---|---|
| Column: | FineLine 100 (Pharmacia, Freiburg, Germany) |
| Column material: | Source RPC, 15 µm |
| | 10 x 12.5 cm (FineLine 100) |
| Flow rate: | 150 ml/min (FineLine 100) |
| Detection: | 280 nm, conductivity, pH |
| Buffer A: | 10 mM HCl |
| Buffer B: | 80% acetonitrile in 10 mM HCl |
| Gradient: | 0-60% buffer B in 5 column volumes |

After charging the individual pH pools, the column is washed with buffer A. During elution, fractions of 200 ml are collected. The fractions are freeze-dried and stored at -20 °C. Fractions 6-8 from pH pool II contained the peptide ALB408-423.

### 4th step: Semi preparative reverse-phase C18 chromatography:

A total of 200 mg (corresponding to 1087 liters of hemofiltrate equivalent amount) of fractions 6-8 from pH pool II was separated through a semipreparative reverse-phase column. Fractions 33 + 34 contained ALB408-423.

### Chromatographic conditions:

| | |
|---|---|
| Column: | 4.7 cm x 30 cm steel column |
| Packing material: | Bakerbond RP-C18, 15-30 µm, 300 Å) |
| Buffer A: | 100% water, 10 mM HCl |
| Buffer B: | 80% acetonitrile, 20% water, 10 mM HCl |
| Gradient: | 0-30% B in 2000 ml |
| Flow rate: | 40 ml/min (pressure: 40 bar) |
| Detection: | 214 nm and 280 nm |

### Chromatographic

| | |
|---|---|
| equipment: | BioCad 250, Perseptive Biosystems |
| Fractions: | 50 ml each from start of gradient (min 10.75) |

### 5th step: Semi preparative reverse-phase C18 chromatography:

Fractions 33 + 34 from the previous chromatographic step were separated through a similar semipreparative reverse-phase column using different mobile phases. Fractions 5 + 6 contained ALB408-423.

### Chromatographic conditions:

| | |
|---|---|
| Column: | 4.7 cm x 30 cm steel column |
| Packing material: | Bakerbond RP-C18, 15-30 µm, 300 Å) |
| Buffer A: | 30% methanol, 70% water, 10 mM HCl |
| Buffer B: | 100% methanol, 10 mM HCl |
| Gradient: | 0-15% B in 40 ml |
| | 15-60% B in 1900 ml |
| Flow rate: | 40 ml/min (pressure: 30 bar) |
| Detection: | 214 nm and 280 nm |

### Chromatographic

| | |
|---|---|
| equipment: | BioCad 250, Perseptive Biosystems |
| Fractions: | 50 ml each from start of gradient (min 9.75) |

### 6th step: Analytical reverse-phase C4 chromatography:

Fractions 5 + 6 from the previous chromatography were separated through an analytical reverse-phase column. Fractions 51 to 57 contained ALB408-423.

### Chromatographic conditions:

| | |
|---|---|
| Column: | 2 cm x 25 cm steel column |
| Packing material: | RP-C4, 5 µm, 100 A, Biotek Silica, Östringen, Germany) |
| Buffer A: | water, 0.1% TFA |
| Buffer B: | 80% acetonitrile, 20% water, 0.1% TFA |
| Gradient: | 0-5% B in 2 min, 5-35% B in 60 min, 35-100% B in 3 min |
| Flow rate: | 7 ml/min |
| Detection: | 214 nm and 280 nm |

### Chromatographic

| | |
|---|---|
| equipment: | Kontron |
| Fractions: | 1 min each from min 1 |

### 7th step: Analytical reverse-phase C18 chromatography:

Fractions 51-57 from the previous chromatography were separated through an analytical reverse-phase column. Fraction 31 contained ALB408-423.

### Chromatographic conditions:

| | |
|---|---|
| Column: | 1 cm x 25 cm steel column |
| Packing material: | RP-C18, 5 µm, 300 Å, Vydac (Hesperia, USA) |
| Buffer A: | water, 0.1% TFA |
| Buffer B: | 80% acetonitrile, 20% water, 0.1% TFA |
| Gradient: | 0-15% B in 5 min, 15-45% B in 60 min, 45-100% B in 1 min |
| Flow rate: | 2 ml/min |
| Detection: | 214 nm and 280 nm |

### Chromatographic

| | |
|---|---|
| equipment: | Kontron |
| Fractions: | 1 min each from min 1 |

The ALB408-423 was contained in fraction 31.

### Example 2

### Chemical synthesis of ALB408-423

The chemical synthesis of ALB408-423 was performed by means of conventional solid-phase synthesis on a peptide synthesizer 9050 (Applied Biosystems) using the known Fmoc chemistry. The peptide obtained was purified by reverse-phase chromatography, and its identity and purity were established by analytical RP-HPLC and by MALDI-MS mass determination (Example 3).

### Example 3

### Mass determinations

The mass determinations of the peptide isolated from hemofiltrate (from fraction 31 of the 7th step in Example 1) and on the chemically synthesized peptides (Examples 2 and 4) were performed on a MALDI mass spectrometer (Voyager DE-Pro). The molecular masses of the peptides were determined to correspond to the following mass figures (MW):

| | |
|---|---|
| ALB408-423, isolated from human hemofiltrate: | 1830.9 Da |
| ALB408-423, chemically synthesized peptide: | 1830.6 Da |

### Sequence determination

The purified native peptide was analyzed by means of MS-MS coupling analysis (ESI-TRAP) supplied by the company PROTEOMEFACTORY AG, Dorotheenstr. 94, 10117 Berlin (Germany), by a data base comparison of the established ESI MS-MS masses by means of the Mascot search engine, which resulted in the following sequence with the highest probability:

### LVRYTKKVPQVSTPTL

### Data base comparison

A further data base comparison with the SwissProt data base shows that the peptide sequence has 100% identity with amino acids 408-423 of the human protein serum albumin (Accession No. NP000468), and the sequence contains the amino acids: LVRYTKKVPQVSTPTL.

### Example 4

### Synthesis of peptides which have the biological activity of ALB408-423.

Various ALB408-423 derivatives containing N or C terminal deletions or amino acid substitutions were chemically synthesized (Table 1). These peptides are peptides obtainable by multiple synthesis which have the biological activity of ALB408-423.

**Table 1**

| | | | | | | | | Da* | SEQ ID NO: |
|---|---|---|---|---|---|---|---|---|---|
| ALB415-423 | | | | | | | VPQVSTPTL | 941 | 1 |
| ALB414-423 | | | | | | KVPQVSTPTL | | 1068 | 2 |
| ALB413-423 | | | | | KKVPQVSTPTL | | | 1196 | 3 |
| ALB412-423 | | | | TKKVPQVSTPTL | | | | 1298 | 4 |
| ALB411-423 | | | YTKKVPQVSTPTL | | | | | 1461 | 5 |
| ALB410-423 | | RYTKKVPQVSTPTL | | | | | | 1618 | 6 |
| ALB409-423 | VRYTKKVPQVSTPTL | | | | | | | 1717 | 7 |
| **ALB408-423** | | **LVRYTKKVPQVSTPTL** | | | | | 1832 | | 8 |
| ALB408-422 | | LVRYTKKVPQVSTPT | | | | | 1720 | | 9 |
| ALB408-421 | | LVRYTKKVPQVSTP | | | | | 1619 | | 10 |
| ALB408-420 | | LVRYTKKVPQVST | | | | | 1522 | | 11 |
| ALB408-419 | | LVRYTKKVPQVS | | | | | 1422 | | 12 |
| ALB408-418 | | LVRYTKKVPQV | | | | | 1334 | | 13 |
| ALB408-417 | | LVRYTKKVPQ | | | | | 1232 | | 14 |
| ALB408-416 | | LVRYTKKVP | | | | | n.d. | | 15 |
| **ALB408-415** | | LVRYTKKV | | | | | 1006 | | 16 |
| ALB408-414 | | LVRYTKK | | | | | | 907 | 17 |
| ALB408-413 | | LVRYTK | | | | | | 779 | 18 |
| ALB407-414 | LLVRYTKK | | | | | | 1025 | | 19 |
| ALB407-419 | LLVRYTKKVPQVS | | | | | | 1536 | | 20 |
| ALB408I-419 | | IVRYTKKVPQVS | | | | | 1421 | | 21 |
| ALB408F-419 | | FVRYTKKVPQVS | | | | | 1454 | | 22 |
| ALB408A-419 | | AVRYTKKVPQVS | | | | | 1378 | | 23 |
| ALB408G-419 | | GVRYTKKVPQVS | | | | | 1366 | | 24 |
| | | IVRWTKKVPQVS | | | | | | | 32 |
| | | IVRWTCKVPQVS | | | | | | | 33 |
| | | IVRWSKKVPCVS | | | | | | | 34 |
| | | IVRWSKKVPQVS | | | | | | | 35 |
| | | IMRWSRKMPCVS | | | | | | | 36 |
| | | ILRWSRKLPCVS | | | | | | | 37 |
| | | ILRWSRKMPCVS | | | | | | | 38 |
| | | ILRWTRKMPCVS | | | | | | | 39 |
| | | ILRWSRKMPCMS | | | | | | | 40 |
| | | ILRWSRKFPCVS | | | | | | | 41 |
| | | ILRWSRKMPCFS | | | | | | | 42 |
| | | ILRWSRKMPQFS | | | | | | | 43 |
| | | IVRWSKKMPQVS | | | | | | | 44 |
| | | LVRYTQKAPQVS | | | | | | | 45 |

| **ALB408-415 variants** | | | | | | | | | |
|---|---|---|---|---|---|---|---|---|---|
| ALB-wt | | LVRYTKKV | | | | | 1006 | | 16 |
| ALB-V415A | | LVRYTKKA | | | | | 978 | | 25 |
| ALB-K414A | | LVRYTKAV | | | | | 949 | | 26 |
| ALB-K413A | | LVRYTAKV | | | | | 949 | | 27 |
| ALB-T412A | | LVRYAKKV | | | | | 976 | | 28 |
| ALB-Y411A | | LVRATKKV | | | | | 914 | | 29 |
| ALB-R410A | | LVAYTKKV | | | | | 921 | | 30 |
| ALB-V409A | | LARYTKKV | | | | | 978 | | 31 |

| | | | | | | | | | |
|---|---|---|---|---|---|---|---|---|---|
| *Da: molecular weight (in Da) | | | | | | | | | |

## Claims

1. A peptide for use in treating and/or preventing central nervous disorders, wherein the peptide is selected from the group consisting of the following:
(i) a peptide having the following amino acid sequence:
Z₁-LVRYTKKVPQVSTPTL-Z₂ (ALB-408);
(ii) peptides obtainable by multiple synthesis which have the biological activity of ALB408-423;
and
(iii) biologically active fragments and/or variants and/or derivatives, especially PEGylated, HESylated, amidated, acetylated, sulfated, phosphorylated and/or glycosylated derivatives, of (i) and (ii);
wherein Z represents a number of from 0 to 10 amino acid residues.

2. The peptide for use according to claim 1, wherein the peptides obtainable by multiple synthesis which have the biological activity of ALB408-423 are the peptides selected from the group consisting of SEQ ID NOs: 1 to 31 and/or the peptides having the general amino acid sequence written in the single letter code
X¹ X² R X⁴ X⁵ X⁶ K X⁸ P X¹⁰ X¹¹ S,
wherein
X¹ = Lor I,
X² = V, M, or L,
X⁴ = Y or W,
X⁵ = T or S,
X⁶ = K, C, R, or Q,
X⁸ = V, M, L, F, or A,
X¹⁰ = Q, or C,
X¹¹ = V, M, or F.

3. The peptide for use according to any one of claims 1 or 2, wherein the peptides obtainable by multiple synthesis which have the biological activity of ALB408-423 are the peptides selected from the group consisting of SEQ ID NOs: 1 to 31 and/or the peptides having the general amino acid sequence written in the single letter code
X¹ V R X⁴ X⁵ X⁶ KV P X¹⁰ V S,
wherein
X¹ = L or I,
X⁴ = Y or W,
X⁵ = T or S,
X⁶ = K or C,
X¹⁰ = Q or C.

4. The peptide for use according to any one of claims 1 to 3, wherein the peptide is selected from the group consisting of a peptide having the following amino acid sequence:
IVRYTKKVPQVS (SEQ ID NO: 21),
and its amidated, acetylated, sulfated, phosphorylated and/or glycosylated derivatives.

5. The peptide for use according to any one of claims 1 to 4, wherein the peptide has the following amino acid sequence:
IVRYTKKVPQVS (SEQ ID NO: 21).

6. The peptide for use according to any one of claims 1 to 5, wherein the central nervous disorders is selected from the group consisting of neurodegeneration, chronic pain, stroke, spinal cord injury, Alzheimer's disease, Bell's palsy, cerebral palsy, epilepsy, motor neurone disease, multiple sclerosis, neurofibromatosis, parkinsons's disease, sciatice or shingles.

7. The peptide for use according to any one of claims 1 to 6, wherein the peptide is administered on an intranasal route.

8. The peptide for use according to any one of claims 1 to 7, wherein the peptide is administered on the intranasal route, three times a day.

9. A pharmaceutical composition for use in treating and/or preventing central nervous disorders, wherein the pharmaceutical composition comprises or consists of
(a) a peptide having the following amino acid sequence:
Z₁-LVRYTKKVPQVSTPTL-Z₂ (ALB-408),
peptides obtainable by multiple synthesis which have the biological activity of ALB408-423, and biologically active fragments and/or variants and/or derivatives, especially PEGylated, HESylated, amidated, acetylated, sulfated, phosphorylated and/or glycosylated derivatives thereof; wherein Z represents a number of from 0 to 10 amino acid residues; and
(b) a pharmaceutically acceptable carrier.

10. The pharmaceutical composition for use according to claim 9, wherein the peptide has the following amino acid sequence:
IVRYTKKVPQVS (SEQ ID NO: 21).

11. The pharmaceutical composition for use according to any one of claims 9 or 10, wherein the concentration of the peptide is between 0.5 mg/mL and 2.0 mg/mL, preferably 1.0 mg/mL.

12. The pharmaceutical composition for use according to any one of claims 9 to 11, suitable for intranasal administration.

13. The pharmaceutical composition for use according to any one of claims 9 to 12, wherein the pharmaceutically acceptable carrier is a sodium chloride solution or a citrate/bicarbonate buffer or a citrate/phosphate buffer.

14. A method for treating central nervous disorders in a subject in need thereof comprising, administering to the subject an effective amount of a peptide, wherein the peptide is selected from the group consisting of the following:
(i) a peptide having the following amino acid sequence:
Z₁-LVRYTKKVPQVSTPTL-Z₂ (ALB-408);
(ii) peptides obtainable by multiple synthesis which have the biological activity of ALB408-423;
and
(iii) biologically active fragments and/or variants and/or derivatives, especially PEGylated, HESylated, amidated, acetylated, sulfated, phosphorylated and/or glycosylated derivatives, of (i) and (ii);
wherein Z represents a number of from 0 to 10 amino acid residues.

15. The method according to claim 14, wherein the peptide is administered intranasal.
